# EUROPEAN PATENT SPECIFICATION

(11) **EP 0 347 885 B1**
(45) Date of publication and mention of the grant of the patent: **18.11.1993**
(21) Application number: 89111305.2
(22) Date of filing: 21.06.1989
(51) Int. Cl.: C07D 229/02

(54) **Perfluorodiaziridines and process for preparing them**
Perfluordiaziridine und Verfahren zu ihrer Herstellung
Perfluorodiaziridines et procédé pour leur préparation

(30) Priority: 22.06.1988 IT 2106388
(43) Date of publication of application: 27.12.1989
(73) Proprietor: AUSIMONT S.p.A., I-20121 Milano (IT)
(72) Inventor: Navarrini, Walter, Dr., I-20010 Boffalora S.T. Milan (IT); Desmarteau, Darryl D., Dr., Clemson South Carolina 29631 (US)
(74) Representative: Barz, Peter, Dr.

(56) References cited:
- JOURNAL OF ORGANIC CHEMISTRY vol. 53, 1988, pages 4443-4447; C. W. BAUKNIGHT et al.: "Reactions of N-Bromodifluoromethanimine"
- JOURNAL OF ORGANIC CHEMISTRY vol. 48, 1983, pages 771-774; SHI-CHING CHANG et al.: "Fluoride-Catalyzed Reactions of Perfluoromethanimine. Novel Chemistry of the Perfluoromethanamine Ion"
- CHEMICAL ABSTRACTS vol. 69, no. 17, 21 October 1968, page 6281, abstract no, 67266j, Columbus, Ohio, USA; W. C. FIRTH: "Reactions of pentafluoroguanidine and tetrafluoroformamidine with alkalimetal fluorides. Fluorine containing diaziridines"

## Description

The present invention relates to novel perfluorodiaziridines and to a process for preparing them.
Perfluorodiaziridines are a not very widely known class or organic compounds.
The known perfluorodiaziridine of formula: 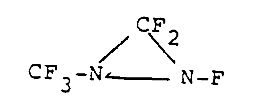
was synthesized by starting from CF₂=N-F and CsF (Shi-Ching Chang and Darryl D. DesMarteau, J. Org. Chem. 1983, 48, 771-774).

An object of the present invention is to provide a novel class of perfluorodiaziridines.
A further object is to provide a process for preparing them.

The first object is achieved by the novel perfluorodiaziridines according to the present invention, having the formula: 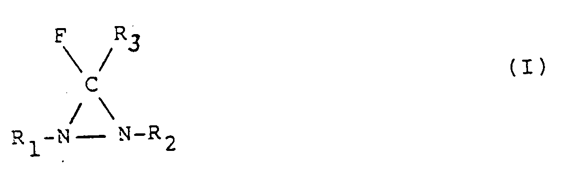
wherein:
- R₁ and R₂,: which may be either equal to or different from each other, represent a perfluoroalkyl group of from 1 to 10 carbon atoms, and
- R₃: represents a fluorine atom, or a perfluoroalkyl group of from 1 to 9 carbon atoms.

The above perfluorodiaziridines may be used as catalysts for the photochemical polymerization of olefinic monomers. They can form complexes with transition metal ions, and are useful as intermediates in the preparation of nitrenes.

Preferably, the groups R₁ and R₂ are perfluoroalkyl groups of from 1 to 3 carbon atoms (i.e. CF₃, C₂F₅ and n- and i-C₃F₇) and R₃ is F, CF₃ or C₂F₅.
The perfluorodiaziridines of the present invention can be prepared by reacting a perfluoroamino-oxaziridine of formula: 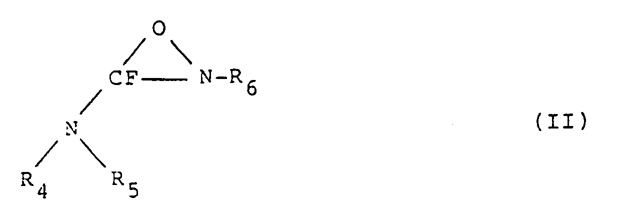
wherein:
- R₄, R₅ and R₆,: which may be either equal to or different from one another, are perfluoroalkyl groups containing from 1 to 10 carbon atoms,
with a source of fluoride ions.
The reaction can schematically be represented as follows: 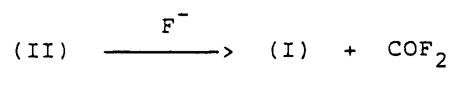
The radical R₃ of (I) originates from the radical R₄ or R₅ of (II), and contains one carbon atom less than the latter, so that R₃ is F when its source radical (R₄ or R₅) is -CF₃.

The R₁ and R₂ radicals of (I) originate from radicals of (II) different from the radical which supplied R₃.
The reaction is commonly carried out at a temperature within the range of from 0 to 120°C.
As the fluoride ion sources, CsF, KF and tetraalkyl-ammonium fluorides are preferably used.

The molar ratio of the fluoride ion source (e.g. CsF) to the perfluoroaminooxaziridine (II) is generally within the range of from 0.1 to 10, and preferably from 1 to 10.

The reaction is preferably carried out in the presence of a dipolar aprotic solvent, such as acetonitrile, the glymes, dimethylformamide and dimethylsulphoxide, or mixtures thereof.

The perfluoroaminooxaziridines (II) and the process for preparing them are disclosed in Italian patent application No. 22,576 A/87, which is incorporated herein by reference.

According to said patent application, the perfluoroaminooxaziridines of formula (II) are obtained by reacting a perfluoroimine of formula: 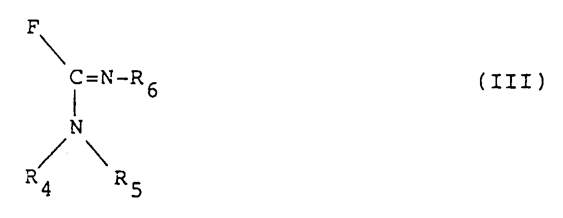
wherein R₄, R₅ and R₆ have the meanings defined above, with H₂O₂ in the presence of a base, in a dipolar aprotic solvent, at a temperature in the range of from -50°C to +50°C.

The following example illustrates the present invention.

### EXAMPLE

A 150 ml glass reactor containing 3 g (5 mmol) of CsF was charged with a perfluoroaminooxaziridine of formula: 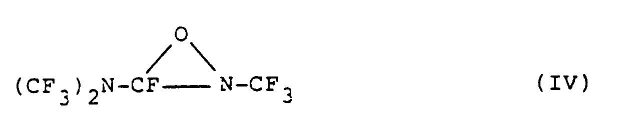
Thereafter the charged reactor was maintained at room temperature for 8 hours.
The crude reaction product was distilled under a pressure of 10⁻³ torr.
The vapours from the distillation flask were passed through two cooling traps maintained at temperatures of -120°C and -196°C, resp.
Inside the trap at -120°C 3 mmol of 1,2-trifluoromethyl-3,3-difluorodiaziridine having the formula: 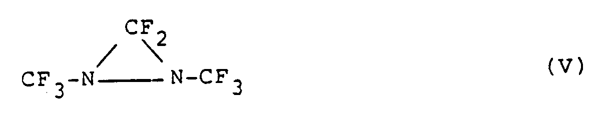
condensed (yield 60%, based on the perfluoroaminooxaziridine used as starting material). Inside the trap at -196°C, a mixture condensed, which mixture predominantly was composed of COF₂, together with some by-products.

The diaziridine of formula (V) was analysed by I.R. spectrum, ¹⁹F-N.M.R. and mass spectrum.
The main absorption bands in the I.R. range were as follows: cm⁻¹ (intensity): 1443 (s); 1317 (vs), 1277 (s), 1245 (vs), 1205 (s) and 996 (m); ("vs" = "very strong", "s" = "strong" and "m" = "medium").
The N.M.R. spectrum (internal reference CFCl₃; solvent CDCl₃) showed the following signals: 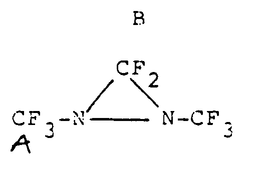
A = (triplet) - 65.6 ppm; J_{AB} = 8 Hz
B = (heptet) - 108.6 ppm; J_{AB} = 8 Hz
The mass spectrum exhibited the following peaks:
M, 216 (1.8%); 69 (100%); 128 (31.3%); 197 (19.1%).

## Claims (Claims for the following Contracting State(s): DE, FR, GB, NL, SE)

1. Perfluorodiaziridines having the formula: 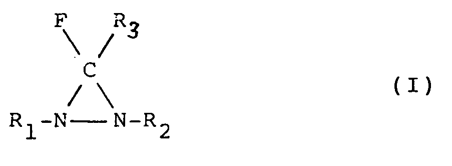 wherein:
R₁ and R₂, the same or different from each other, represent a perfluoroalkyl group having 1 to 10 carbon atoms; and
R₃ represents a fluorine atom or a perfluoroalkyl group having 1 to 9 carbon atoms.

2. Perfluorodiaziridines according to claim 1, wherein R₁ and R₂ are perfluoroalkyl groups of from 1 to 3 carbon atoms, and R₃ is a fluorine atom, or a perfluoroalkyl group of 1 or 2 carbon atoms,

3. 1,2-Trifluoromethyl-3,3-difluorodiaziridine of formula

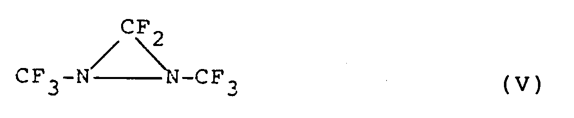

4. Process for preparing the perfluorodiaziridines according to claim 1, characterized in that a perfluoroamino-oxaziridine of formula: 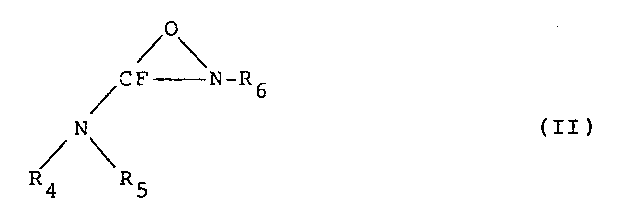 wherein:
R₄, R₅ and R₆, the same or different from each other, are perfluoroalkyl groups containing from 1 to 10 carbon atoms,
is reacted with a fluoride ion source.

5. Process according to claim 4, characterized in that the reaction is carried out at a temperature of from 0 to 120°C.

6. Process according to any one of claims 4 and 5, characterized in that the source of fluoride ions is selected from CsF, KF and tetraalkyl-ammonium fluorides.

7. Process according to one or bore of claims 4 to 6, characterized in that the reaction is carried out in the presence of a dipolar aprotic solvent.

8. Process according to claim 7, characterized in that the dipolar aprotic solvent is selected from acetonitrile, the glymes, dimethylformamide, dimethylsulphoxide or mixtures thereof.

9. Use of the compounds according to any one of claims 1 to 3 as catalysts for the photochemical polymerization of olefins.

10. Use of the compounds according to any one of claims 1 to 3 for the preparation of nitrenes.

## Claims (Claims for the following Contracting State(s): ES)

1. Process for preparing perfluorodiaziridines having the formula: 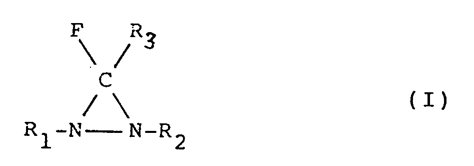 wherein:
R₁ and R₂, the same or different from each other, represent a perfluoroalkyl group having 1 to 10 carbon atoms; and
R₃ represents a fluorine atom, or a perfluoroalkyl group having 1 to 9 carbon atoms,
characterized in that a perfluoroamino-oxaziridine of formula: 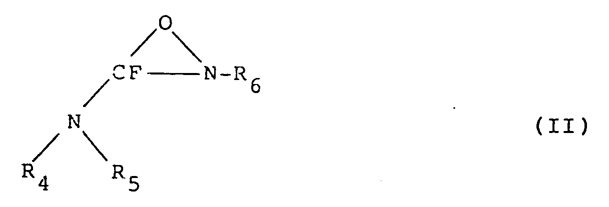 wherein:
R₄, R₅ and R₆, the same or different from each other, are perfluoroalkyl groups containing from 1 to 10 carbon atoms,
is reacted with a fluoride ion source.

2. Process according to claim 1, characterized in that the reaction is carried out at a temperature of from 0 to 120°C.

3. Process according to any one of claims 1 and 2, characterized in that the source of fluoride ions is selected from CsF, KF and tetraalkyl-ammonium fluorides.

4. Process according to one or more of claims 1 to 3, characterized in that the reaction is carried out in the presence of a dipolar aprotic solvent.

5. Process according to claim 4, characterized in that the dipolar aprotic solvent is selected from acetonitrile, the glymes, dimethylformamide, dimethylsulphoxide or mixtures thereof.

6. Process according to any one of claims 1 to 5 wherein perfluorodiaziridines are prepared wherein R₁ and R₂ are perfluoroalkyl groups of from 1 to 3 carbon atoms, and R₃ is a fluorine atom, or a perfluoroalkyl group of 1 or 2 carbon atoms.

7. Process according to any one of claims 1 to 5 wherein 1,2-trifluoromethyl-3,3-difluorodiaziridine of formula 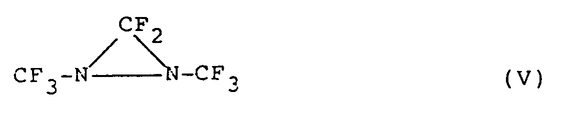 is prepared.

8. Process for the photochemical polymerization of olefins wherein compounds prepared according to any one of claims 1 to 7 are used as catalysts.

9. Process for the preparation of nitrenes wherein compounds prepared according to any one of claims 1 to 7 are used.

## Patentansprüche (Patentansprüche für folgende(n) Vertragsstaat(en): DE, FR, GB, NL, SE)

1. Perfluordiaziridine der allgemeinen Formel: 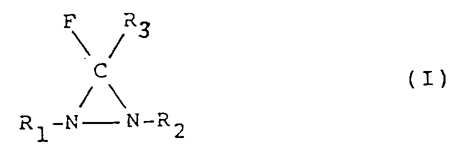 worin:
R₁ und R₂, gleich oder verschieden voneinander, eine Perfluoralkylgruppe mit 1 bis 10 Kohlenstoffatomen darstellen; und
R₃ für ein Fluoratom oder eine Perfluoralkylgruppe mit 1 bis 9 Kohlenstoffatomen steht.

2. Perfluordiaziridine nach Anspruch 1, worin R₁ und R₂ Perfluoralkylgruppen mit 1 bis 3 Kohlenstoffatomen sind und R₃ für ein Fluoratom oder eine Perfluoralkylgruppe mit 1 oder 2 Kohlenstoffatomen steht.

3. 1,2-Trifluormethyl-3,3-difluordiaziridine der Formel

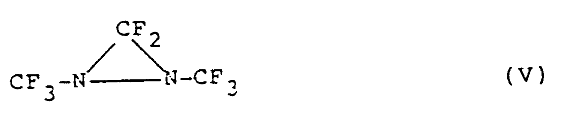

4. Verfahren zur Herstellung der Perfluordiaziridine nach Anspruch 1, dadurch gekennzeichnet, daß ein Perfluoramino-Oxaziridin der Formel 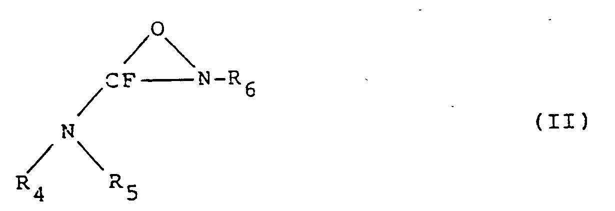 worin:
R₄, R₅ und R₆, gleich oder verschieden voneinander, Perfluoralkylgruppen, die 1 bis 10 Kohlenstoffatome enthalten, sind,
mit einer Fluoridionen-Quelle umgesetzt wird.

5. Verfahren nach Anspruch 4, dadurch gekennzeichnet, daß die Umsetzung bei einer Temperatur von 0 bis 120°C durchgeführt wird.

6. Verfahren nach irgendeinem der Ansprüche 4 und 5, dadurch gekennzeichnet, daß die Fluoridionen-Quelle aus CsF, KF und Tetraalkylammoniumfluoriden ausgewählt wird.

7. Verfahren nach einem oder mehreren der Ansprüche 4 bis 6, dadurch gekennzeichnet, daß die Umsetzung in Anwesenheit eines dipolaren aprotischen Lösungsmittels durchgeführt wird.

8. Verfahren nach Anspruch 7, dadurch gekennzeichnet, daß das dipolare aprotische Lösungsmittel aus Acetonitril, den Glymen, Dimethylformamid, Dimethylsulfoxid oder Mischungen davon ausgewählt wird.

9. Verwendung der Verbindungen nach irgendeinem der Ansprüche 1 bis 3 als Katalysatoren für die photochemische Polymerisation von Olefinen.

10. Verwendung der Verbindungen nach irgendeinem der Ansprüche 1 bis 3 für die Herstellung von Nitrenen.

## Patentansprüche (Patentansprüche für folgende(n) Vertragsstaat(en): ES)

1. Verfahren zur Herstellung von Perfluordiaziridinen mit der Formel 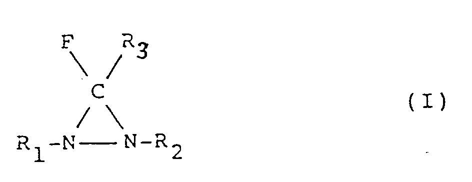 worin:
R₁ und R₂, gleich oder verschieden voneinander, für eine Perfluoralkylgruppe mit 1 bis 10 Kohlenstoffatomen stehen; und
R₃ ein Fluoratom oder eine Perfluoralkylgruppe mit 1 bis 9 Kohlenstoffatomen darstellt,
dadurch gekennzeichnet, daß ein Perfluoramino-Oxaziridin der Formel 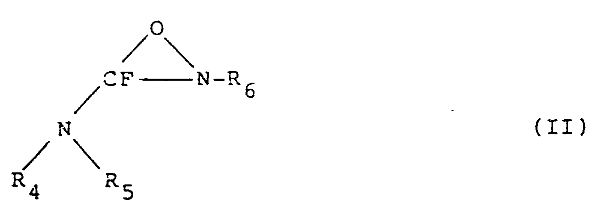 worin:
R₄, R₅ und R₆, gleich oder verschieden voneinander, Perfluoralkylgruppen, die 1 bis 10 Kohlenstoffatome enthalten, darstellen,
mit einer Fluoridionen-Quelle umgesetzt wird.

2. Verfahren nach Anspruch 1, dadurch gekennzeichnet, daß die Umsetzung bei einer Temperatur von 0 bis 120°C durchgeführt wird.

3. Verfahren nach irgendeinem der Ansprüche 1 und 2, dadurch gekennzeichnet, daß die Fluoridionen-Quelle aus CsF, KF und Tetraalkylammoniumfluoriden ausgewählt wird.

4. Verfahren nach irgendeinem der Ansprüche 1 bis 3, dadurch gekennzeichnet, daß die Umsetzung in Anwesenheit eines dipolaren aprotischen Lösungsmittels durchgeführt wird.

5. Verfahren nach Anspruch 4, dadurch gekennzeichnet, daß das dipolare aprotische Lösungsmittel aus Acetonitril, den Glymen, pimethylformamid, Dimethylsulfoxid oder Mischungen davon ausgewählt wird.

6. Verfahren nach irgendeinem der Ansprüche 1 bis 5, in welchem Perfluordiaziridine hergestellt werden, worin R₁ und R₂ Perfluoralkylgruppen mit 1 bis 3 Kohlenstoffatomen sind und R₃ für ein Fluoratom oder eine Perfluoralkylgruppe mit 1 oder 2 Kohlenstoffatomen steht.

7. Verfahren nach irgendeinem der Ansprüche 1 bis 5, in welchem 1,2-Trifluormethyl-3,3-difluordiaziridin der Formel 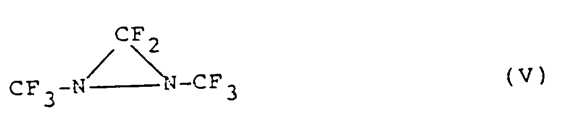 hergestellt wird.

8. Verfahren zur photochemischen Polymerisation von Olefinen, in welchem Verbindungen, die nach irgendeinem der Ansprüche 1 bis 7 hergestellt wurden, als Katalysatoren eingesetzt werden.

9. Verfahren zur Herstellung von Nitrenen, in welchem Verbindungen, die gemäß irgendeinem der Ansprüche 1 bis 7 hergestellt wurden, eingesetzt werden.

## Revendications (Revendications pour l'(les) Etat(s) contractant(s) suivant(s): DE, FR, GB, NL, SE)

1. Perfluorodiaziridines répondant à la formule: 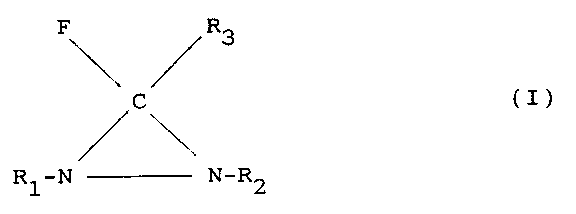 dans laquelle:
R₁ et R₂, identiques ou différents l'un de l'autre, représentent un groupe perfluoroalkyle renfermant de 1 à 10 atomes de carbone, et
R₃ représente un atome de fluor ou un groupe perfluoroalkyle renfermant de 1 à 9 atomes de carbone.

2. Perfluorodiaziridines selon la revendication 1, caractérisées en ce que R₁ et R₂ sont des groupes perfluoroalkyle renfermant de 1 à 3 atomes de carbone et R₃ est un atome de fluor ou un groupe perfluoroalkyle renfermant de 1 à 2 atomes de carbone.

3. 1,2-trifluorométhyl-3,3-difluorodiaziridine de formule:

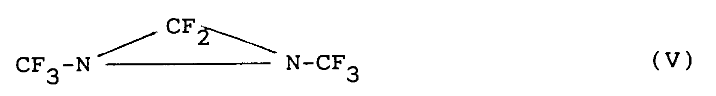

4. Procédé de préparation des perfluorodiaziridines selon la revendication 1, caractérisé en ce que l'on fait réagir avec une source d'ions fluorure une perfluoroaminooxaziridine de formule: 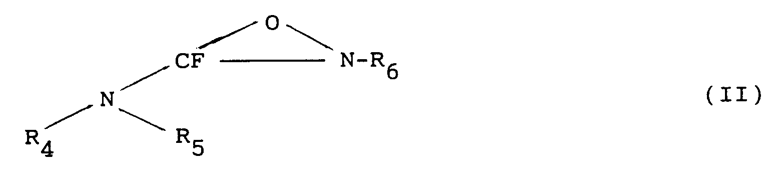 dans laquelle:
R₄, R₅ et R₆, identiques ou différents l'un de l'autre, sont des groupes perfluoroalkyle renfermant de 1 à 10 atomes de carbone.

5. Procédé selon la revendication 4, caractérisé en ce que la réaction est mise en oeuvre à une température comprise entre 0 et 120°C.

6. Procédé selon l'une quelconque des revendications 4 et 5, caractérisé en ce que la source d'ions fluorure est choisie parmi CsF, KF et les fluorures de tétraalkylammonium.

7. Procédé selon l'une quelconque des revendications 4 à 6, caractérisé en ce que la réaction est mise en oeuvre en présence d'un solvant aprotique dipolaire.

8. Procédé selon la revendication 7, caractérisé en ce que le solvant aprotique dipolaire est choisi parmi l'acétonitrile, les glymes, le diméthylformamide, le diméthylsulfoxyde ou leurs mélanges.

9. Utilisation des dérivés selon l'une quelconque des revendications 1 à 3 comme catalyseurs pour la polymérisation photochimique des oléfines.

10. Utilisation des dérivés selon l'une quelconque des revendications 1 à 3 pour la préparation des nitrènes.

## Revendications (Revendications pour l'(les) Etat(s) contractant(s) suivant(s): ES)

1. Procédé de préparation de perfluorodiaziridines répondant à la formule: 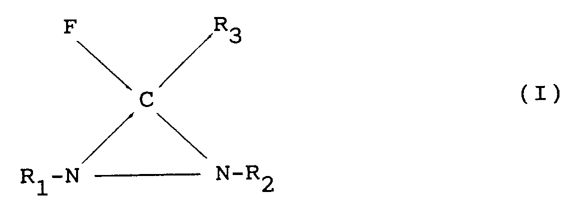 dans laquelle:
R₁ et R₂, identiques ou différents l'un de l'autre, représentent un groupe perfluoroalkyle renfermant de 1 à 10 atomes de carbone, et
R₃ représente un atome de fluor ou un groupe perfluoroalkyle renfermant de 1 à 9 atomes de carbone,
caractérisé en ce que l'on fait réagir avec une source d'ions fluorure une perfluoroaminooxaziridine de formule: 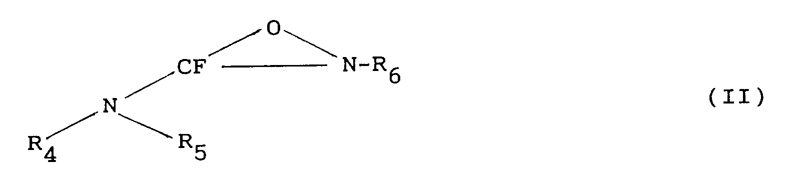 dans laquelle:
R₄, R₅ et R₆, identiques ou différents les uns des autres, sont des groupes perfluoroalkyle renfermant de 1 à 10 atomes de carbone.

2. Procédé selon la revendication 1, caractérisé en ce que la réaction est mise en oeuvre à une température comprise entre 0 et 120°C.

3. Procédé selon l'une quelconque des revendications 1 et 2, caractérisé en ce que la source d'ions fluorure est choisie parmi CsF, KF et les fluorures de tétraalkylammonium.

4. Procédé selon l'une quelconque des revendications 1 à 3, caractérisé en ce que la réaction est mise en oeuvre en présence d'un solvant aprotique dipolaire.

5. Procédé selon la revendication 4, caractérisé en ce que le solvant aprotique dipolaire est choisi parmi l'acétonitrile, les glymes, le diméthylformamide, le diméthylsulfoxyde ou leurs mélanges.

6. Procédé selon l'une quelconque des revendications 1 à 5, caractérisé en ce que l'on préfère des fluorodiazinidines dans lesquelles R₁ et R₂ sont des groupes perfluoroalkyles renfermant de 1 à 3 atomes de carbone, et R₃ est un atome de fluore ou un groupe perfluoroalkyle renfermant 1 ou 2 atomes de carbone.

7. Procédé selon l'une quelconque des revendications 1 à 5, caractérisé en ce que l'on prépare une 1,2-trifluorométhyl-2-3-difluorodiazinidine de formule V:

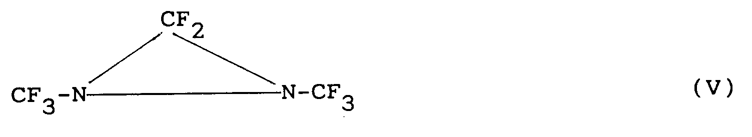

8. Procédé de polymérisation photochimique d'oléfines caractérisé en ce que les dérivés préparés selon l'une quelconque des revendications 1 à 7 sont utilisés en tant que catalyseurs.

9. Procédé de préparation de nitrènes caractérisé en ce que les dérivés préparés selon l'une quelconque des revendications 1 à 7 sont utilisés.
